# EUROPÄISCHE PATENTANMELDUNG

(11) **EP 1 234 612 A2**
(43) Veröffentlichungstag der Anmeldung: **28.08.2002**
(21) Anmeldenummer: 02001179.7
(22) Anmeldetag: 29.01.2002
(51) Int. Cl.: B01J 19/24, B01J 35/04, B01J 35/00, B01J 37/02, B01J 37/34, F28D 9/00

(54) **Reaktor zur Durchführung von katalysierten Reaktionen**

(30) Priorität: 21.02.2001 DE 10108380
(71) Anmelder: DEG Intense Technologies & Services GmbH, 82049 Pullach (DE)
(72) Erfinder: Heisel, Michael, Dr., 82049 Pullach (DE); Daun, Klaus, 45289 Essen (DE)
(74) Vertreter: Best, Michael, Dr.

(57) **Zusammenfassung**

Die Erfindung betrifft einen Reaktor zur Durchführung katalysierter chemischer Reaktionen mit einem Wärmetauscher umfassend voneinander durch Thermobleche getrennte Reaktions- und Wärmetransporträume, dadurch gekennzeichnet, dass der Katalysator in Form einer dünnen Schicht auf zumindest einem Teil der Fläche der Thermobleche aufgebracht ist, welche dem Reaktionsraum zugewandt ist.

## Beschreibung

Die Erfindung betrifft einen Reaktor zur Durchführung von katalysierten Reaktionen insbesondere zur Durchführung von Reaktionen, bei denen entweder sehr viel Wärme anfällt und daher abgeführt werden muss oder von Reaktionen, bei denen es aus Sicherheitsgründen und/oder zur Vermeidung von Nebenreaktionen zu keiner Wärmeentwicklung kommen darf bzw. sich entwickelnde Wärme möglichst gut und umgehend abgeführt werden muss. Insbesondere ist der erfindungsgemäße Reaktor in einem Verfahren zur Herstellung von Ethylenoxid aus Ethylen und Sauerstoff in der Gasphase einsetzbar.

Reaktoren zur Durchführung katalysierter endo- oder exothermer Reaktionen sind im Stand der Technik in verschiedenen Ausführungsformen bekannt. Bei katalysierten Prozessen im industriellen Maßstab werden im Regelfall rieselfähige Katalysatorpartikel verwendet, die zwischen zwei Wandungen gebracht werden. Die Größe der Katalysatorpartikel liegt häufig im Bereich von einigen Millimetern Kantenlänge. Auf der anderen Seite der Wandungen befindet sich ein Medium geeigneter Wärmekapazität, welches die Reaktionswärme der Reaktion ab- oder zuführt. Somit werden derartige Reaktoren in Reaktionsräume und Wärmetransporträume unterteilt. Häufig befinden sich in einem Reaktor mehrere Reaktionsräume, die durch die Wärmetransporträume voneinander getrennt sind. Da die Reaktionen, die in den Reaktoren ablaufen, häufig unter erhöhtem Druck und bei Temperaturen oberhalb der Umgebungstemperatur durchgeführt werden, ist eine ausreichend stabile Bauweise erforderlich. Reaktoren zur Durchführung derartiger Reaktionen werden zum Beispiel in U.S.1,850,398, U.S.3,127,247 und U.S.4,544,544 beschrieben. Die Aufteilung der Reaktoren in mehrere Reaktionsräume hat z.B. den Vorteil, dass durch diese Kompartimentierung die Einzelvolumina der unabhängigen Reaktionsräume vergleichsweise klein gehalten werden. Dies ist insofern von Interesse, als dass bei exotherm ablaufenden chemischen Reaktionen bei nicht ausreichender Wärmeabfuhr die Gefahr besteht, dass die Reaktionen einen unkontrollierten Verlauf nehmen. Dies kann im Extremfall zu Verpuffungen oder Explosionen führen. Eine unzureichende Wärmeabfuhr bei exothermen Reaktionen, wie auch eine unzureichende Wärmezufuhr bei endothermen Reaktionen, resultiert in einer heterogenen Temperaturverteilung innerhalb des Reaktors. Da sehr häufig in katalytischen Prozessen verschiedene Reaktionen bei verschiedenen Temperaturen ablaufen, kann sich eine derartige heterogene Temperaturverteilung in einem Verlust an Selektivität niederschlagen. Daher ist eine nach Möglichkeit gleichmäßige Temperaturverteilung, im Idealfall isotherme Reaktionsführung erstrebenswert. Auf diese Weise können die Reaktionen exakt kontrolliert und die Bildung von Nebenprodukten unterdrückt werden. Schon eine Effizienzsteigerung der Reaktionsführung im Bereich von einigen Zehntel Prozent ist im Regelfall bei industriellen Prozessen, für welche die Reaktoren eingesetzt werden, mit erheblichen ökonomischen Vorteilen verbunden.

Gerade auch bei großindustriellen Verfahren muss aus Sicherheitsgründen unbedingt verhindert werden, dass es zu Verpuffungen oder Explosionen kommen kann.

Das Problem einer möglicherweise uneinheitlichen Wärmeabfuhr bei katalytischen Reaktionen, insbesondere bei Verwendung von plattentauschergekühlten Reaktoren, wird in der U.S.1,850,398 beschrieben. Zur Lösung dieser Probleme schlägt diese Druckschrift spezielle Wärmeaustauscher vor, die mit einem "doppelten Gegenstrom" arbeiten. Dieses Verfahren reicht aber nicht aus, um eine möglichst isotherme Reaktionsführung zu gewährleisten und die vorstehenden Probleme ausreichend zu lösen.

In EP-A-1 002 571 werden gekühlte Trennwände mit Hilfe von Metallplatten/metailischen Baueinheiten gebildet, wobei zur Kühlung in den Metallplatten/metaiiischen Baueinheiten Hohl- oder Zwischenräume in Form von Kanälen zur Aufnahme und zum Durchleiten eines Kühlmediums angeordnet sind. Die Katalysatorpartikel werden zwischen zwei derartige Kühlelemente gebracht. In DE-C-19 754 185 sind Wärmetauscherplatten beschrieben, welche als vom Kühlmedium durchströmte Thermobleche ausgebildet sind, die aus zumindest zwei Blechplatten aus Stahl bestehen und die in den Katalysatorräumen an vorgegebenen Punkten unter Bildung von Strömungskanälen zusammengefügt sind. Die Katalysatorpartikel werden auch hier zwischen die Kühlelemente gebracht.

Derartige Reaktoren haben den Nachteil, dass im Lauf der Verwendung des Reaktors die Katalysatorpartikel verkleben können und dann nur mit großem Aufwand aus dem Reaktor wieder zu entfernen sind. Außerdem können sich beim Befüllen des Reaktors Katalysatorkörner gegen die Thermobleche verkeilen, so dass sich darunter Hohlräume bilden, in denen kein Katalysator ist. Solche Hohlräume werden wegen des geringeren Druckverlusts bevorzugt von Reaktionsgas durchströmt, wobei jedoch kein Umsatz stattfindet. Verluste an Umsatz sind selbstverständlich unerwünscht. Zudem sind Katalysatorbrücken bei der Entleerung des Reaktors nur schwer zu entfernen. Weiterhin beansprucht das Einfüllen der Katalysatorpartikel, dass zwischen den Thermoblechen ein Abstand eingehalten wird, welcher mindestens dem räumlichen Ausmaß eines Katalysatorpartikels entspricht. Dies hat zur Folge, dass die Kühl-/Heizfläche pro Reaktionsraumvolumen kaum über einen Wert von 150m²/m³ hinausgehen kann. Für besonders stark exotherme Reaktionen oder solche Reaktionen, bei denen eine genaue Temperatureinhaltung erforderlich ist, kann dies zu gering sein, um auftretende Wärme sicher, vollständig und schnell abzuführen bzw. Temperaturschwankungen im Reaktionsraum zu vermeiden. Bei der Befüllung des Reaktors mit den Katalysatorpartikeln kommt es auch häufig zur mechanischen Beanspruchung des Katalysators. Dadurch wird Katalysatorstaub gebildet, welcher sich vorwiegend am Austritt aus dem Katalysatorbett sammelt. Unvorteilhafterweise ist im Regelfall diese Stelle gerade derjenige Ort, an dem auch die Kühl-/Heizfläche endet. Die Kühlleistung an dieser Stelle des Reaktors ist somit geringer. Da Staub eine deutlich größere Oberfläche als die Katalysatorpartikel gleicher Masse hat, ist er häufig katalytisch besonders aktiv, was dazu führt, dass gerade von solchem Staub die Gefahr des Durchgehens der Reaktion ausgeht.

Dies ist insbesondere nachteilig bei der Umsetzung von Ethylen und Sauerstoff zu Ethylenoxid, da es hierbei zu Folgereaktionen kommen kann, bei denen das Ethylenoxid unter einer stark exothermen Reaktion mit dem vorhandenen Sauerstoff verbrennt.

In der Patentschrift U.S. 3,528,783 sind Reaktoren offenbart, bei denen rieselfähige Katalysatorpartikel zwischen Kühlelemente gebracht werden. Handelt es sich bei dem Katalysator um ein Metall oder um ein Derivat eines Metalls, welches geschweißt oder durch vergleichbare Verfahren an der den Reaktor begrenzenden Metallfläche befestigt werden kann, so ist es auch gemäß einer Ausführungsform möglich, dass eine gewellte Matrix aus diesem Material in den Reaktionsraum eingebracht wird. Diese Matrix besteht dann in massiver Ausgestaltung aus diesem Katalysator. Als Beispiele für geeignete Metalle werden Nickel, Silber und Eisen erwähnt.

In der Offenlegungsschrift DE 42 14 579 ist ein Reaktor offenbart, in dessen Gehäuse Fluidpfade bildende Strukturen derart eingebracht werden, daß sich kanalförmige Strukturen ergeben und diese abschnittweise katalytisch wirkende Bereiche besitzen. Durch diese Anordnung werden bei stets gleicher Strömungsrichtung des Fluids Zonen unterschiedlicher Temperatur erreicht. Diese heterogene Temperaturzonenverteilung ist jedoch für die Katalyse von selektiven chemischen Reaktionen besonders ungünstig, da diese effizient nur unter isothermen Bedingungen durchgeführt werden können.

Bei den bekannten Reaktoren zur Durchführung katalysierter chemischer Reaktionen ist in der Regel auch eine schnelle Ableitung der in dem Reaktionsraum auftretenden Wärme nur schwer möglich, da die Wärme zunächst zu den Thermoblechen geleitet werden muss. Auf der Seite der Wärmetransporträume liegen die Wärmeübergangszahlen erheblich über den Wärmeübergangszahlen innerhalb des Reaktionsraums. Im Prinzip wären daher die Thermobleche durchaus in der Lage, erheblich mehr Wärme abzuleiten, als Ihnen im Reaktionsraum aufgrund der geringen Wärmeübergangszahlen des Reaktionsmediums zugeleitet wird.

Insbesondere für großtechnische industrielle Anwendungen besteht derzeit noch immer ein Bedarf an Reaktoren zur katalytischen Reaktionsführung, welche die Nachteile der Reaktoren des Standes der Technik vermeiden. Diese Aufgabe wird durch den Gegenstand der Patentansprüche gelöst.

Die Erfindung betrifft einen Reaktor zur Durchführung katalysierter chemischer Reaktionen mit einem Wärmetauscher umfassend voneinander durch Thermobleche getrennte Reaktions- und Wärmetransporträume, dadurch gekennzeichnet, dass der Katalysator in Form einer dünnen Schicht auf zumindest einem Teil der Fläche der Thermobleche aufgebracht ist, welche dem Reaktionsraum zugewandt ist. Bevorzugt sind zumindest 50% der Fläche der Thermobleche, welche dem Reaktionsraum zugewandt ist, mit Katalysator beschichtet, stärker bevorzugt zumindest 90%, am meisten bevorzugt ist die Fläche vollständig mit Katalysator beschichtet.

Die feste Verbindung zwischen katalytisch aktivem Material und Reaktorgehäuse ist beispielsweise zur Aufarbeitung von Abgasen bei Katalysatoren im Automobilbau bekannt, jedoch nicht in großtechnischen Anlagen zur katalytischen Synthese von chemischen Produkten. Die erfindungsgemäßen Reaktoren können bei einer spezifisch vorgegebenen Temperatur nahezu isotherm betrieben werden und eignen sich daher hervorragend für industrielle Zwecke. Der Abstand zwischen den Thermoblechen kann sehr klein gewählt werden, sodass das Reaktorvolumen zwischen den Platten gering wird. Dies vermindert sehr deutlich die Gefahr, die von einem unkontrollierten Durchgehen der Reaktion in dem Reaktionsraum ausgehen kann. Diese wird außerdem minimiert, da durch einen geringen Abstand zwischen den Thermoblechen und damit einem kleinen Reaktionsraumvolumen bezogen auf die Wärmetauscherfläche gewährleistet wird, dass bei exothermen Reaktionen nur verhältnismäßig wenig Wärme anfällt, die darüber hinaus besonders gut abgeführt werden kann. Lokale Temperaturinhomogenitäten (hot spots) können vermieden werden, da die Temperatur des Thermobleches nur wenige Grad von der Temperatur des Kühl-/Heizmediums abweicht und die Verteilung des Katalysators im Reaktionsraum gleichförmig ist.

Bei der erfindungsgemäßen Ausgestaltung der Reaktoren zur Durchführung katalysierter chemischer Reaktionen findet die Umsetzung in unmittelbarer Nähe der dem Reaktionsraum zugewandten Wände des Thermobleches statt und hier entsteht im Wesentlichen die Reaktionswärme. Ein Wärmetransport von der Mitte des Reaktionsraums zu den Thermoblechen mit den geringen Wärmeübergangszahlen entfällt, sodass bei den erfindungsgemäßen Reaktoren die Wärme wesentlich besser abgeleitet werden kann als bei den Reaktoren des Standes der Technik. Da sich damit bei den erfindungsgemäßen Reaktoren die Temperatur des Thermoblechs allenfalls um wenige Grad von der Temperatur des Kühlheizmediums unterscheidet, können die im Stand der Technik auftretenden vielfach gefürchteten "hot spots" im Reaktionsraum damit bei den erfindungsgemäßen Reaktoren zuverlässig vermieden werden.

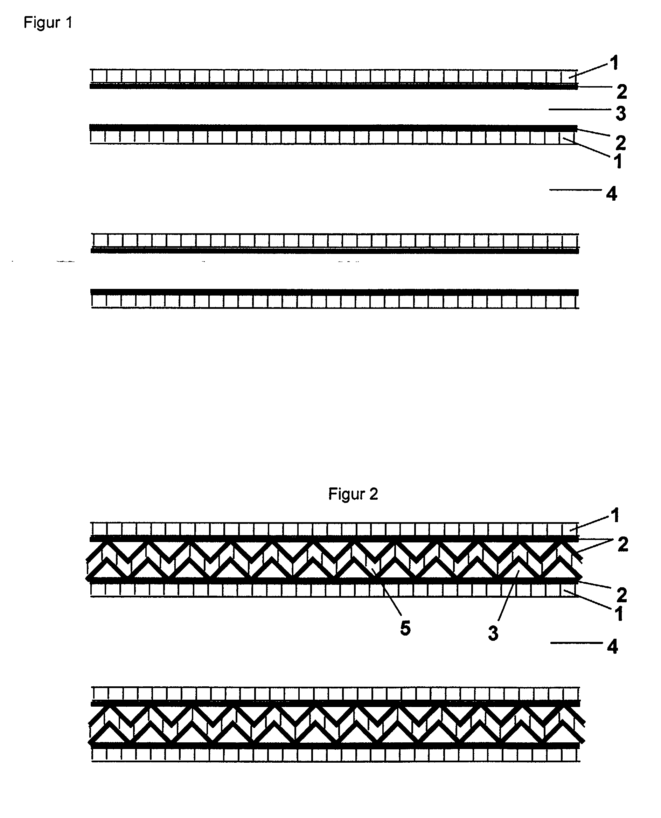

Bevorzugte Ausführungsformen der erfindungsgemäßen Reaktoren sind in den Figuren 1 bis 4 schematisch abgebildet:
Figur 1 zeigt einen Schnitt durch ein Reaktorkompartiment. Die Thermobleche (1) sind auf den zum Reaktionsraum (3) zugewandten Seiten mit dem Katalysator (2) beschichtet. Der Wärmetransportraum (4) befindet sich jeweils auf der anderen Seite der Thermobleche.
Figur 2 zeigt eine Ausführungsform, bei der zusätzlich im Reaktionsraum (3) ein gewelltes Modul (5) eingebracht ist, welches auf seiner Oberfläche ebenfalls mit Katalysator (2) beschichtet ist. Der Reaktionsraum (3) hat in dieser Ausführungsform angenähert einen dreieckigen Querschnitt.
Figur 3 zeigt eine bevorzugte Ausführungsform, bei der zwei Thermobleche (1) an bestimmten Punkten fest miteinander verbunden und kissenartig gewölbt vorliegen. Die Wölbung der Bleche ist zur Veranschaulichung stark ausgeprägt dargestellt. Bezugszeichen 3 bezeichnet den Reaktionsraum, Bezugszeichen 4 den Wärmetransportraum. Die dem Reaktionsraum (3) zugewandte Fläche der Thermobleche (1) ist mit Katalysator beschichtet. Die Pfeile geben die Durchflußrichtung von Reaktions- bzw. Kühlmedium an. Diese Ausführungsform entspricht in ihrem konstruktionellen Aufbau den Reaktoren der DE-C 19 754 185.
Figur 4 zeigt weitere Ausführungsformen, wobei stets nur die den Reaktionsraum (3) begrenzenden Thermobleche (1), welche eine unterschiedliche Dicke aufweisen können, abgebildet sind.

Da die Oberfläche der Thermobleche und damit der Katalysatoren nahezu gleich der Kühl-/Heiztemperatur des Kühl-/Heizmediums ist und auftretende Wärme direkt abgeführt wird, kann man sehr genau die gewünschte Temperatur für die Reaktion einstellen und einhalten. Das gilt insbesondere, wenn als Kühlmittel, wie üblicherweise eine gut wärmeleitende Flüssigkeit, wie z.B. verdampfendes Kesselspeisewasser verwendet wird. Darüber hinaus muss nicht wie in konventionellen Reaktoren ein Kompromiss zwischen Eintritts- und Austrittstemperatur gewählt werden, sondern die optimale Reaktionstemperatur kann im gesamten Reaktor eingestellt werden. Die genaue Temperaturkontrolle erhöht die Selektivität der Reaktion.

Durch die Bauweise der erfindungsgemäßen Reaktoren kann das Befüllen mit Edukten und das Abführen von Produkten sicher und einfach erfolgen. Der Betrieb erfolgt sicher und zuverlässig, da u.a. die By-pass-Strömung entfällt, welche bei Schüttgutreaktoren beobachtet wird. Der Druckverlust innerhalb der Reaktorkompartimente ist gering. Die Verteilung des Reaktionsmediums über den Reaktorquerschnitt kann besonders gleichmäßig erfolgen.

Der Abstand zwischen den beschichteten Thermoblechen kann bis auf ca. 1 mm reduziert werden. Dies bedeutet dann eine Kühl-/Heizfläche von bis zu ca. 500 m²/m³. Mit einer derart großen Wärmetauscherfläche lassen sich nahezu beliebig stark exotherme Reaktionen durchführen, ohne dass dabei die Gefahr besteht, dass aufgrund der Wärmeeinwirkung der Reaktionen sich der Katalysator oder das Reaktionsvolumen übermäßig erhitzt und folglich die Reaktion zu schnell wird und unkontrollierte Zustände erreicht. Je größer das Verhältnis zwischen Katalysatorfläche und Reaktionsraumvolumen ist, desto besser lassen sich die durchgeführten Reaktionen kontrollieren. Die erfindungsgemäßen Reaktoren erreichen hier Werte, die mehr als doppelt so groß sind wie die entsprechenden Werte für die Reaktoren im Stand der Technik. Mit den erfindungsgemäßen Reaktoren können daher auch Reaktionen im industriellen Maßstab durchgeführt werden, die sich durch eine derart große Wärmetönung auszeichnen, dass sie bisher lediglich mit Reaktoren kleinerer Bauart aus dem Stand der Technik realisiert werden konnten. Durch den geringen Abstand der Thermobleche voneinander wird erreicht, dass die im entsprechenden Reaktionsraum befindliche Substanzmenge vergleichsweise gering ist. Sollte dennoch einmal eine unkontrollierte Reaktion stattfinden, so ist demnach die von der geringen Substanzmenge ausgehende Gefahr nur gering, zumal die auftretende Wärme sehr schnell abgeführt werden kann.

Ein weiterer Vorteil der erfindungsgemäßen Reaktoren betrifft das Verhältnis zwischen Katalysatorfläche und Katalysatormasse. Während bei den Reaktoren im Stand der Technik, bei denen die Katalysatorpartikel zwischen die Thermobleche geschüttet oder anderweitig eingebracht wird, im Regelfall Werte zwischen 50 und 100 m² pro Tonne Katalysator erreicht werden, beträgt der entsprechende Wert bei den erfindungsgemäßen Reaktoren mehr als 10⁶, bevorzugt mehr als 10⁷ m² pro Tonne Katalysator z.B. bis zu 30 000 000 m² pro Tonne Katalysator, bei sehr geringer Schichtdicke.

Der erfindungsgemäße Reaktor eignet sich insbesondere zur Durchführung von Reaktionen mit starker Wärmetönung und zwar sowohl von exothermen Reaktionen als auch von endothermen Reaktionen. Beispiele möglicher Reaktionen, die vorteilhafterweise in dem erfindungsgemäßen Reaktor durchgeführt werden können, sind die Epoxidation von Olefinen wie z.B. die Herstellung von Ethylenoxid, die CO-Konvertierung zur H₂-Gewinnung, die Direktoxidation von H₂S zu Elementarschwefel, die Clausreaktion, die Hydrierung von Kohlenwasserstoffen, insbesondere die selektive Hydrierung von Kohlenwasserstoffen, wie die Hydrierung von Acetylen zu Ethylen, die Oxidation von SO₂ zu SO₃, die Methanolsynthese, die Methansynthese, die Fischer-Tropsch-Synthese, die NH₃-Synthese, die Herstellung von Acrylsäure, Maleinsäure, Phthalsäure, Essigsäure, Acrolein, Methacrolein, Methacrylsäure, Glyoxal, selektive Oxidation von Alkanen, z.B. Cyclohexan, Hydrierung von Nitrogruppen, aromatischer Kerne, Estern, Säuren und Pyrolysebenzin, Dehydrierung und Oxidehydrierungen, speziell zu Styrol etc. Die meisten dieser Reaktionen sind exotherm. Prinzipiell ist der Reaktor jedoch ebenso geeignet, um darin endotherme Reaktionen durchzuführen.

Bei den Reaktionen, welche im erfindungsgemäßen Reaktor durchgeführt werden können, handelt es sich bevorzugt um Gasphasenreaktionen. Es ist aber auch möglich, Flüssigphasenreaktionen durchzuführen, bei denen auch Gase oder Feststoffe in gelöster Form vorliegen können. In speziellen Fällen können auch verflüssigte Gase zum Einsatz kommen, welche im Extremfall in überkritischem Zustand vorliegen können. Die Durchflussgeschwindigkeit des Reaktionsmediums kann dabei in weiten Grenzen variiert werden. Bevorzugt liegt sie für Gase im Bereich von 1 bis 60 m/s (gerechnet bei Normalzustand, 1 bar, 25°C), besonders bevorzugt im Bereich 10 bis 30 m/s. Für Flüssigkeiten liegt sie im Bereich 0,2 bis 5 m/s, besonders bevorzugt im Bereich 0,5 bis 2,5 m/s.

Der Katalysator kann auf verschiedene Weise auf die Thermobleche aufgebracht werden. Als Methoden kommen die bekannten Methoden im Stand der Technik in Frage, als Beispiel seien CVD (chemical vapor deposition), PVD (physical vapor deposition), Sputtern und Reaktivsputtern, galvanometrische Methoden und oberflächensensitive chemische Reaktionen, oder Wash Coat am Thermoblech erwähnt. Der Katalysator kann dabei direkt auf das Thermoblech aufgetragen werden, es ist aber auch möglich, dass zunächst eine oder mehrere Zwischenschichten aufgetragen werden.

Ein weiterer Vorteil des erfindungsgemäßen Reaktors kann darin gesehen werden, dass keine Katalysatorpartikel in den Reaktionsraum eingefüllt werden müssen, was zur Bildung von Katalysatorstaub führen würde und im Fall von dünn mit Katalysator beschichteten Trägerpartikeln zu einer Beschädigung der Katalysatorschicht führen würde.

Die Schicht des Katalysators in den erfindungsgemäßen Reaktoren kann im Bereich von 1 bis 1000 µm liegen, bevorzugt ist eine Schichtdicke von weniger als 50 µm.

Falls es aus technischen Gründen schwierig sein sollte, den Katalysator direkt auf die Thermobleche aufzubringen, insbesondere wenn diese aus dem bevorzugten Material Edelstahl sind, ist es selbstverständlich möglich, eine geeignete Zwischenschicht bzw. einen Träger vorzusehen, die das Aufbringen des Katalysators erleichtert und/oder das Haften des Katalysators auf dem Thermoblech verbessert. Geeignete Zwischenschichten sind im Stand der Technik bekannt, beispielsweise sind Al₂O₃, ZrO₂, Silikate, insbesondere auch Zeolithe geeignet.

Als Katalysatoren eignen sich alle im Stand der Technik bekannten Stoffe, die auf ein Thermoblech oder eine geeignete Zwischenschicht aufgetragen werden können und für die Katalyse einer entsprechenden chemischen Reaktion geeignet sind. Insbesondere handelt es sich dabei um Metalle oder Salze von Metallen, hierbei insbesondere um Metalloxide. Bevorzugte Metalle sind Übergangsmetalle und Lanthanoide, aber auch Metalle der Hauptgruppen und ihre Derivate z.B. Salze können geeignet sein. Bevorzugt sind die Metalle Ti, Zr, Hf, V, Nb, Ta, Cr, Mo, W, Mn, Re, Fe, Rh, Os, Co, Ru, Ir, Ni, Pd, Pt, Cu, Ag, Au, Zn, Cd, Hg und deren Oxide, in Reinform oder als Mischungen, Legierungen oder intermetallische Phasen.

Bevorzugt bestehen die Thermobleche aus Metall, besonders bevorzugt aus Stahl. Die Thermobleche können z.B. eine Stärke von 0,3 bis 3 mm aufweisen. Bevorzugt ist eine Stärke von 0,8 bis 2 mm. Dabei kann das Aufbringen des Katalysators auf die Oberfläche der Thermobleche bewirken, dass deren Korrosion unterdrückt wird. Dies ist insbesondere von Vorteil, wenn mögliche Korrosionsprodukte wie z.B. Rost die Wirkung des Katalysators inhibieren würden.

Das Kühl- oder Heizmedium kann in verschiedenen Richtungen durch den Reaktor transportiert werden. Ein Durchfluss im Gegenstrom oder in Durchflussrichtung des Reaktors ist möglich, auch gemischte Formen sind realisierbar. Als Kühlmedien eignen sich bevorzugt Flüssigkeiten mit hoher Wärmekapazität, im Falle von exothermen Reaktionen ist Wasser ein bevorzugtes Medium. Besonders bevorzugt wird Kesselspeisewasser verwendet. Bei Temperaturen unterhalb von 0°C ist es auch möglich, beispielsweise niedere Alkohole einzusetzen. Um das Strömungsverhalten des Mediums im Wärmetransportraum zu verbessern, können Trennwände verschiedener Ausgestaltung zusätzlich eingebaut werden.

Die erfindungsgemäßen Reaktoren bestehen aus einem äußeren, bevorzugt röhrenförmigen Druckmantel, in den vorgefertigte Module aus parallel angeordneten Thermoblechen eingebracht werden können. Die einzelnen Module können verschiedene Größe haben, sie werden bevorzugt derart in den Druckmantel eingebracht, dass sie den Reaktorinnenraum möglichst gut ausfüllen. Es können auch mehrere Module hintereinander im Reaktor angeordnet sein. Die Reaktoren können z.B. in waagerechter oder senkrechter Anordnung betrieben werden.

Die modulare Bauweise hat den Vorteil, dass zur Regeneration des Katalysators ein schneller Austausch mit Reservemodulen erfolgen kann. Die Wartungszeiten des erfindungsgemäßen Reaktors sind somit sehr kurz. Die aus dem Reaktor entnommenen Module können dann erneut mit Katalysator beschichtet oder auf anderem Wege regeneriert werden. Das Befüllen mit rieselfähigen Katalysatorpartikeln und die damit verbundenen Probleme entfallen. Der Katalysator kann auch in situ regeneriert werden, indem er je nach verwendetem Katalysator oxidierend oder reduzierend, beispielsweise hydrierend behandelt wird. In der bevorzugten Ausführungsform, in der die Thermobleche aus Edelstahl gebildet werden, kann eine Behandlung bei hohen Temperaturen und unter aggressiven Bedingungen erfolgen. Insbesondere können zur Regenerierung des Katalysators saure Medien eingesetzt werden. Die Temperaturen können bis an die Grenze derjenigen Temperatur gehen, welche für die Thermobleche gerade noch zulässig ist.

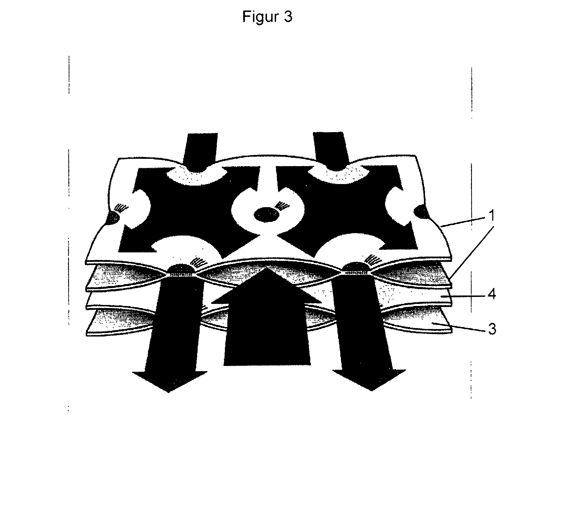

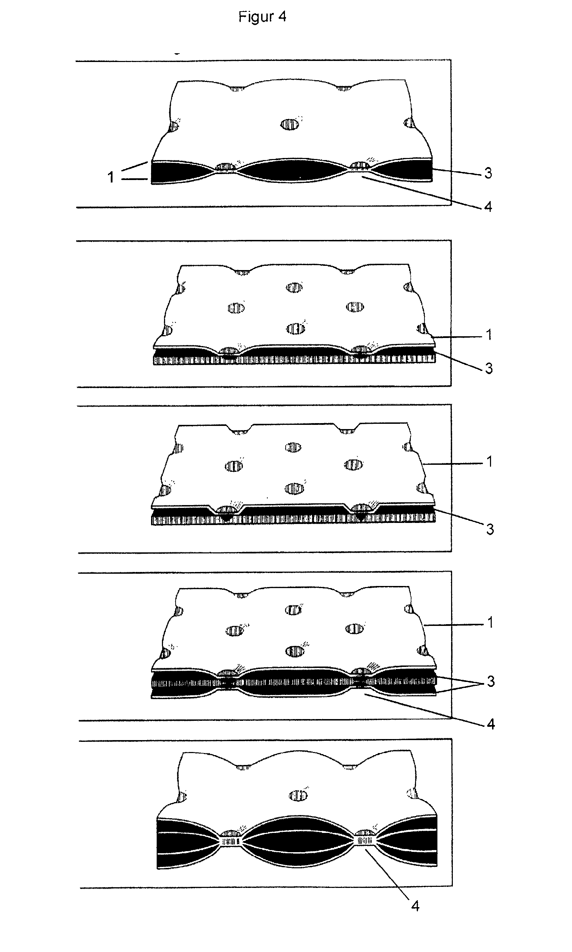

Die Thermobleche können konzentrisch oder parallel angeordnet sein, bei besonderer Druckbeanspruchung sind auch spezielle Formgestaltungen möglich. In einer bevorzugten Ausführungsform werden Thermobleche verwendet, die an bestimmten Punkten miteinander verbunden sind. Bei dieser Ausführungsform ist es auch möglich, dass bei der Herstellung zwischen die Platten für kurze Zeit eine Hydraulikflüssigkeit gepresst wurde, mit einem Druck, der um Größenordnungen größer ist, als der zu erwartende Druck während der chemischen Reaktion im Reaktor. Durch die Hydraulikflüssigkeit werden die Platten bauchig auseinandergedrückt, sodass kissenartige Strukturen entstehen (Figur 3 und 4).

Damit ähnelt in einer bevorzugten Ausführungsform der erfindungsgemäße Reaktor dem in der DE-C-19 754 185 beschriebenen Reaktor, wobei allerdings der Reaktorraum nicht wie in der DE-C-19 754 185 beschrieben mit einem teilchenförmigen Katalysator gefüllt wird, sondern erfindungsgemäß die Thermobleche zumindest teilweise durch den Katalysator beschichtet sind. Auch ist es nicht notwendig, wie in der DE-C-19 754 185 beschrieben, dass die Thermobleche unmittelbar miteinander verbunden sind, sondern die Thermobleche können auch über kurze Stege miteinander verbunden sein, sodass die kissenförmige Struktur nicht derart ausgeprägt ist wie in der DE-C-19 754 185. Insgesamt kann aber zur genauen Ausgestaltung des Reaktors auf diese Druckschrift verwiesen werden.

In einer weiteren bevorzugten Ausführungsform wird ein Reaktor wie er in der EP-A-1 002 571 beschrieben ist, verwendet, wobei allerdings wiederum nicht, wie in der EP-A-1 002 571 beschrieben, die Reaktorräume mit partikelförmigem Katalysator gefüllt sind, sondern erfindungsgemäß die Thermobleche zumindest teilweise mit Katalysator beschichtet sind. Gegenüber den in der EP-A-1 002 571 beschriebenen Reaktoren können daher die gekühlten Trennwände enger zusammenliegen, sodass die Reaktionsräume ein kleineres Volumen haben, wie es vorstehend ausführlich beschrieben wurde. Ansonsten kann zur Ausgestaltung des Reaktors, insbesondere zur Ausgestaltung des Kühlsystems des Reaktors, voll inhaltlich auf die EP-A-1 002 571 verwiesen werden.

Die Thermobleche können verschiedene Abstandsweiten voneinander haben. Die Abstände der Thermobleche in den Reaktionsräumen sind bevorzugt von 1 bis 50 mm. Besonders bevorzugt sind Abstände von 1 bis 20 mm auf der Seite der Reaktionsräume. Je nach Länge des Reaktors und Umfang des äußeren Druckmantels kann das Gesamtvolumen des Reaktionsraumes im Reaktor bis mehrere hundert m³ betragen. Dabei ist ein wesentlicher Vorteil der Thermobleche, dass sie zu Modulen zusammengefasst in den Reaktorbehälter eingebracht werden können. Dadurch ist es möglich, den drucktragenden Mantel des Reaktors auch auf der Baustelle zu fertigen, und damit ist man nicht durch die Transportmaße in der Größe des Reaktors eingeschränkt.

Die Reaktionen können im allgemeinen bei einem Druck zwischen 1 und 300 bar durchgeführt werden. Reaktionstemperaturen liegen im allgemeinen zwischen 20°C und 400°C.

Einige Beispiele sind:
- Die selektive Acetylenhydrierung zu Ethylen läuft z.B. bei ca. 70°C und ca. 25 bar ab.
- Bei der Methanolsynthese wird die Temperatur typisch bei 220°C, der Druck im Bereich 60 bis 150 bar eingestellt.
- Bei der Ammoniaksynthese kommen Temperaturen von ca. 350°C und Drücke von ca. 300 bar zur Anwendung.

In einer bevorzugten Ausführungsform befinden sich im Reaktionsraum zwischen den mit Katalysator beschichteten Thermoblechen weitere Module aus einem Träger, auf dessen Oberfläche der Katalysator ebenfalls aufgebracht ist. Diese Module können an bestimmten Punkten fest mit den Wandungen der Reaktionsräume verbunden sein. Das Trägermaterial kann bevorzugt aus Keramik oder Metall hergestellt sein, zur Erhöhung der Formstabilität kann es gebogen sein, beispielsweise in Zickzack-Linien geprägt. Bevorzugt sind auch die sogenannten Honey-Comb-Katalysatoren, welche auf Keramiken aufgebracht sind.

Die den Reaktionsraum begrenzenden, mit Katalysator beschichteten Thermobleche werden bevorzugt möglichst nahe an das in den Reaktionsraum zusätzlich eingebrachte Modul angepasst, um einen möglichst effektiven Wärmeübergang zum Wärmetransportraum zu gewährleisten. Je nach Geometrie des Moduls kann es möglich sein, dass der Reaktionsraum zwischen den Thermoblechen durch das Modul in weitere, kleinere, in sich geschlossene Kompartimente unterteilt wird.

In einer bevorzugten Ausführungsform befinden sich im Reaktionsraum zwischen den mit Katalysator beschichteten Thermoblechen keine derartigen Module, so daß der Katalysator ausschließlich auf den Thermoblechen, gegebenenfalls über eine geeignete Zwischenschicht, aufgebracht ist.

Die erfindungsgemäßen Reaktoren können auf verschiedene Weise hergestellt werden. Die Beschichtung der Thermobleche mit dem Katalysator erfolgt bevorzugt durch CVD, PVD, Sputtern, Reaktivsputtern, galvanometrische Methoden oder oberflächensensitive chemische Reaktionen am Thermoblech. In einem bevorzugten Verfahren zur Herstellung der Reaktorkompartimente werden entsprechend mit dem Katalysator beschichtete Blechelemente parallel übereinander gebracht und an den seitlichen, rechtwinkligen Begrenzungen fest und gasdicht miteinander verbunden. Durch wiederholtes Stapeln derartiger Blechelemente entstehen Module, in denen sich in paralleler Anordnung Reaktions- und Wärmetransporträume abwechseln. Derartige Herstellungsverfahren sind im Prinzip im Stand der Technik bekannt.

Diese Module werden dann in einen äußeren Druckmantel gebracht.

Als Beispiel sei die Herstellung von Ethylenoxid aus der Reaktion von Ethylen mit Sauerstoff beschrieben. Ein konventioneller Reaktor für die Herstellung von ca. 75 000 Tonnen pro Jahr hat einen Durchmesser von 5,4 m und eine Höhe über alles von 13,5 m. Er enthält Geradrohre, in die der Katalysator eingefüllt wird. Die Rohre sind von außen mit Kesselspeisewasser gekühlt. Sie enden in dicken Rohrböden, die die Wärmespannungen aller Rohre aufnehmen müssen, die sich auf einem anderen Temperaturniveau befinden als der Druckmantel des Reaktors. Zur Kühlung der Reaktion muß das Kesselspeisewasser ca. 40 bar Druck haben. Um diesen Druck aufnehmen zu können, muß der Druckmantel ca. 100 mm dick sein. Das Gesamtgewicht eines solchen Reaktors liegt bei ca. 500 Tonnen.

Im Vergleich dazu hat ein erfindungsgemäßer Reaktor einen Durchmesser von 4,0 m und eine Höhe über alles von 6,50 m. Er enthält ein Vielfaches der Wärmetauscherfläche des konventionellen Reaktors. Jedoch entfallen die dicken Rohrböden zur Aufnahme der Katalysatorrohre, die bei Thermoblechen nicht erforderlich sind. Da sich das Kesselspeisewasser innerhalb der Thermobleche befindet, muß der Mantel des Reaktors nur den Gasdruck von 25 bar aufnehmen, nicht den höheren Druck des Kesselspeisewassers. Somit kann auch der Mantel dünner ausgeführt werden. In Summe wiegt deshalb der erfindungsgemäße Reaktor nur ca. 220 Tonnen, also weniger als die Hälfte des konventionellen Reaktors.

Die Thermobleche sind aus Edelstahl hergestellt. Der Katalysator ist eine dünne Silberschicht, die im wash-coat-Verfahren über eine Zwischenschicht aus Al₂O₃ auf das Thermoblech aufgebracht wurde. Zwischenschicht und Katalysator haben zusammen eine Dicke von weniger als 0,5 mm.

Der wesentliche Vorteil ist jedoch, dass der Reaktor wegen der wesentlich höheren Kühlfläche ein Durchgehen der Reaktion zuverlässig verhindert. Zwischen den Thermoblechen befinden sich nur sehr kleine Reaktionsvolumina von ca. 20 Liter pro Pass. Selbst wenn die Reaktion durchgehen sollte, was der Reaktor ohnehin sehr zuverlässig verhindert, wäre nur ein kleines Volumen daran beteiligt und damit die freiwerdende Energie gering. Eine Gefahr für die Umgebung besteht nicht. Damit kann folglich das Produkt in einem kleineren und leichteren Reaktor mit höherer Sicherheit gegenüber dem Stand der Technik hergestellt werden.

## Patentansprüche

1. Reaktor zur Durchführung katalysierter chemischer Reaktionen mit einem Wärmetauscher, umfassend voneinander durch Thermobleche getrennte Reaktions- und Wärmetransporträume, **dadurch gekennzeichnet, dass** der Katalysator in Form einer dünnen Schicht auf zumindest einem Teil der gesamten Fläche der Thermobleche aufgebracht ist, welche dem Reaktionsraum zugewandt ist.

2. Reaktor nach Anspruch 1, **dadurch gekennzeichnet, dass** der Katalysator in Form einer dünnen Schicht auf zumindest 50% der Fläche der Thermobleche aufgebracht ist, welche dem Reaktionsraum zugewandt ist.

3. Reaktor nach Anspruch 1 oder 2, **dadurch gekennzeichnet, dass** der Katalysator eine Schichtdicke von weniger als 50 µm aufweist.

4. Reaktor nach einem der vorstehenden Ansprüche, **dadurch gekennzeichnet, dass** die Thermobleche auf der Seite der Reaktionsräume einen Abstand von 1 bis 50 mm aufweisen.

5. Reaktor nach einem der vorstehenden Ansprüche, **dadurch gekennzeichnet, dass** die Thermobleche im wesentlichen aus Stahl bestehen.

6. Reaktor nach einem der vorstehenden Ansprüche, **dadurch gekennzeichnet, dass** es sich bei dem Katalysator um ein Oxid oder die metallische Form eines Nebengruppenelementes handelt.

7. Reaktor nach Anspruch 6, **dadurch gekennzeichnet, dass** zwischen Katalysator und Thermoblech eine Zwischenschicht aus Al₂O₃ aufgebracht ist.

8. Reaktor nach Anspruch 6 oder 7, **dadurch gekennzeichnet, dass** es sich bei dem Katalysator um Ti, Zr, Hf, V, Nb, Ta, Cr, Mo, W, Mn, Re, Fe, Rh, Os, Co, Ru, Ir, Ni, Pd, Pt, Cu, Ag, Au, Zn, Cd, Hg oder Oxide davon handelt, in Reinform oder als Mischungen, Legierungen oder intermetallische Phasen.

9. Reaktor nach einem der vorstehenden Ansprüche, **dadurch gekennzeichnet, dass** die Wärmetauscherfläche, die mit katalytisch wirksamem Material beschichtet ist, zumindest eine Million Quadratmeter pro Tonne Katalysator beträgt.

10. Reaktor nach einem der vorstehenden Ansprüche, bei dem die Thermobleche im Reaktionsraum an vorgegebenen Punkten unter Bildung von Strömungskanälen zusammengefügt sind.

11. Verfahren zur Herstellung eines Reaktors nach einem der vorstehenden Ansprüche, **dadurch gekennzeichnet, dass** der Katalysator durch CVD, PVD, Sputtern, Reaktivsputtern, galvanometrische Methoden als Wash Coat oder oberflächensensitive chemische Reaktionen am Thermoblech auf dieses aufgebracht wird.

12. Verwendung eines Reaktors nach einem der Ansprüche 1 bis 10 zur Durchführung einer katalysierten exothermen Reaktion.

13. Verwendung eines Reaktors nach Anspruch 12, wobei es sich bei der katalysierten exothermen Reaktion um die Umsetzung von Sauerstoff mit Ethylen zu Ethylenoxid in der Gasphase handelt.

14. Verwendung nach Anspruch 12 oder 13, wobei der Katalysator nachdem er in seiner Leistung nachgelassen hat durch saure Medien und/oder erhöhte Temperatur regeneriert wird.
